# EUROPÄISCHE PATENTANMELDUNG

(11) **EP 4 793 343 A1**
(43) Veröffentlichungstag der Anmeldung: **19.08.2026**
(21) Anmeldenummer: 25157334.1
(22) Anmeldetag: 12.02.2025
(51) Int. Cl.: C12M 1/32, C12M 1/12

(54) **VORRICHTUNG ZUR KULTIVIERUNG ZELLBIOLOGISCHER PROBEN IN EINEM HÖHENVSTELBAREN KULTIVIERUNGSEINSATZ**

(71) Anmelder: ibidi GmbH, 82166 Gräfelfing (DE)
(72) Erfinder: Huber, Nina, 85221 Dachau (DE); Lubins, Georg, 80637 München (DE); Pham, Trung, 81543 München (DE); Horn, Elias, 90537 Feucht (DE); Schwarz, Jan, 72511 Bingen (DE); Zantl, Roman, 82166 Gräfelfing (DE); Hönig, Juliane, 79713 Bad Säckingen (DE)
(74) Vertreter: Grünecker Patent- und Rechtsanwälte PartG mbB

(57) **Zusammenfassung**

Die vorliegende Erfindung betrifft eine Vorrichtung zur Kultivierung zellbiologischer Proben in einem Kultivierungseinsatz umfassend: ein Grundelement, in dem eine Vertiefung mit einem Boden ausgebildet ist, wobei der Kultivierungseinsatz in der Vertiefung angeordnet werden kann, und ein Positionierungselement, das eine erste Aufnahmeposition und eine zweite Aufnahmeposition für den Kultivierungseinsatz bereitstellt, wobei der Kultivierungseinsatz in der ersten Aufnahmeposition und in der zweiten Aufnahmeposition am Positionierungselement angeordnet werden kann, sodass der Kultivierungseinsatz in der Vertiefung angeordnet ist, und wobei in der ersten Aufnahmeposition ein Abstand zwischen dem Boden der Vertiefung und einem Boden des Kultivierungseinsatzes kleiner ist als in der zweiten Aufnahmeposition.

## Beschreibung

Die vorliegende Erfindung betrifft eine Vorrichtung zur Kultivierung zellbiologischer Proben.

Heutzutage werden für zelluläre Grenzflächenexperimente wie Co-Kulturen und Transmigrationsexperimente Transwell-Systeme oder Membraneinsatz-Systeme verwendet. Dabei basieren fast alle Membraneinsätze, auch Kultivierungseinsätze genannt, auf demselben Prinzip: Eine poröse Membran dient als Boden des Membraneinsatzes, wodurch ein Topf mit einem porösen, für Zellen und Moleküle durchlässigen, Boden gebildet wird. Dieser Topf kann in ein Reservoir eingesetzt werden, das Nährflüssigkeit enthält. Die poröse Membran dient dann als Gerüst oder Substrat für die Zellanhaftung, wodurch Zellen ein- oder mehrschichtige Strukturen auf der Membran ausbilden können, und lässt gleichzeitig Nährstoffe aus der Nährflüssigkeit hindurch diffundieren, um die Zellen mit Nährstoffen zu versorgen. Das Reservoir, in dem der Membraneinsatz angeordnet ist, muss genügend Platz für eine Nährlösung unter und um den Membraneinsatz bieten, da die Kultivierung von lebenden Zellen unterhalb des Bodens einen ausreichenden Nährstoffaustausch benötigt. Deshalb darf der Abstand zwischen dem Boden des Reservoirs und der porösen Membran bei der Kultivierung nicht unter einen bestimmten Schwellwert sinken.

Gleichzeitig schränkt dieser Schwellwert für den Abstand zum Boden des Reservoirs die Mikroskopierbarkeit der Zellen auf der porösen Membran mit Standardobjektiven erheblich ein, wenn die Zellen mittels inverser Mikroskopie durch den Boden hindurch untersucht werden sollen. Eine Möglichkeit zur Umgehung dieses Problems besteht darin, die poröse Membran aus dem Membraneinsatz zu entfernen und auf einem Mikroskopie-Deckglas zu positionieren. Während dies für die Endpunktanalyse einzelner Proben möglich ist, ist eine Prozesskontrolle und Automatisierung der Mikroskopie auf diese Weise nicht realisierbar.

Daher besteht die Notwendigkeit einer einfachen und anwenderfreundlichen Vorrichtung, die eine geeignete Anordnung für einen Wechsel der Position des Membraneinsatzes zwischen einer idealen Position für die Kultivierung, also eine ausreichende Zuführung von Nährlösung unterhalb der porösen Membran durch einen Mindestabstand zwischen Reservoirboden und poröser Membran, und einer idealen Position für die Bildgebung, also mit einem möglichst kleinen Abstand zwischen dem Objektiv und den Zellen bzw. Proben, bietet. Mit den bisherigen Lösungen kann eine solche Vorrichtung nicht zufriedenstellend bewerkstelligt werden.

Vor diesem Hintergrund ist es eine Aufgabe der vorliegenden Erfindung, eine Vorrichtung zur Kultivierung zellbiologischer Proben bereitzustellen, die einen einfachen und anwenderfreundlichen Mechanismus für einen Wechsel zwischen einer Position des Membraneinsatzes für die Kultivierung und einer Position für die Mikroskopie enthält.

Diese Aufgabe wird durch den Gegenstand von Anspruch 1 gelöst. Weiterbildungen sind durch die abhängigen Ansprüche gegeben.

Erfindungsgemäß wird eine Vorrichtung zur Kultivierung zellbiologischer Proben bereitgestellt, umfassend: ein Grundelement, in dem eine Vertiefung mit einem Boden ausgebildet ist, wobei der Kultivierungseinsatz in der Vertiefung angeordnet werden kann, und ein Positionierungselement, das eine erste Aufnahmeposition und eine zweite Aufnahmeposition für den Kultivierungseinsatz bereitstellt, wobei der Kultivierungseinsatz in der ersten Aufnahmeposition und in der zweiten Aufnahmeposition am Positionierungselement angeordnet werden kann, sodass der Kultivierungseinsatz in der Vertiefung angeordnet ist, und wobei in der ersten Aufnahmeposition ein Abstand zwischen dem Boden der Vertiefung und einem Boden des Kultivierungseinsatzes kleiner ist als in der zweiten Aufnahmeposition.

Damit stellt die Vorrichtung zwei unterschiedliche Aufnahmepositionen für den Kultivierungseinsatz bereit, wobei dieser in beiden Positionen in der Vertiefung angeordnet ist. Somit ragt der Kultivierungseinsatz in beiden Aufnahmepositionen in die Vertiefung hinein. Die erste Aufnahmeposition zeichnet sich durch einen geringeren Abstand zwischen dem Boden des Kultivierungseinsatzes und dem Boden der Vertiefung als die zweite Aufnahmeposition aus. Folglich ist die erste Aufnahmeposition vor allem für die Mikroskopie geeignet und die zweite Aufnahmeposition für die Kultivierung vorgesehen. Diese beiden Aufnahmepositionen werden von dem Positionierungselement bereitgestellt. Ein Wechsel zwischen den beiden Aufnahmepositionen kann also mit Hilfe des Positionierungselements erfolgen, wobei es insbesondere nicht nötig ist, den Kultivierungseinsatz aus der Vertiefung zu heben bzw. zu entfernen. Dadurch wird der Wechsel zwischen den beiden Aufnahmepositionen vereinfacht und anwendungsfreundlicher.

Die erste Aufnahmeposition kann eine Position sein, in der der Abstand zwischen dem Boden des Kultivierungseinsatzes und dem Boden der Vertiefung null ist. Dies bedeutet, dass der Boden des Kultivierungseinsatzes auf dem Boden der Vertiefung aufliegt. Alternativ kann der Abstand zwischen dem Boden des Kultivierungseinsatzes und dem Boden der Vertiefung weniger als 1 mm betragen. In der zweiten Aufnahmeposition ist der Abstand zwischen dem Boden der Vertiefung und dem Boden des Kultivierungseinsatzes größer als in der ersten Aufnahmeposition.

Dadurch kann eine Nährlösung zwischen diesem Abstand gut strömen und einen Nährstoffaustausch durch die poröse Membran sicherstellen. In der zweiten Aufnahmeposition kann der Abstand 1 mm oder mehr, insbesondere auch 2 mm oder mehr, oder sogar 3 mm oder mehr betragen.

Der Kultivierungseinsatz kann auch als Membraneinsatz oder Membran-Insert bezeichnet werden.

Der Boden der Vertiefung kann transparent sein. Dies ermöglicht inverse Mikroskopie durch den Boden hindurch. Proben, die sich in dem Kultivierungseinsatz befinden können so einfach untersucht werden. Der Boden der Vertiefung kann insbesondere im sichtbaren Wellenlängenbereich transparent sein. Stattdessen oder zusätzlich kann auch Transparenz im nah-infraroten Wellenlängenbereich gegeben sein (z.B. zwischen 800 nm und 1500 nm). Zusammen mit dem Abstand zwischen dem Boden des Kultivierungseinsatzes und dem Boden der Vertiefung von weniger als 1 mm in der ersten Aufnahmeposition kann bei inverser Mikroskopie durch den Boden hindurch eine hohe Auflösung erreicht werden.

Der Boden der Vertiefung kann eine Dicke zwischen 100 µm und 2,0 mm, insbesondere zwischen 160 µm und 190 µm, weiter insbesondere zwischen 165 µm und 175 µm, beispielsweise 170 µm, aufweisen. Dadurch ist in der ersten Aufnahmeposition der Abstand zwischen der einem Boden des Kultivierungseinsatzes und einem Objektiv möglichst gering, was eine hohe Auflösung erlaubt. Der Boden der Vertiefung kann ein Glas oder einen Kunststoff wie zum Beispiel eine Polymerfolie umfassen oder sein. Der Boden der Vertiefung kann die optischen Eigenschaften, beispielsweise hinsichtlich Doppelbrechung und Autofluoreszenz, eines Deckglases aufweisen (wie D 263 Schott Glas, Nr. 1.5H). Die Vertiefung kann die Form eines Kegelstumpfes oder eines Zylinders aufweisen. Ein Querschnitt der Vertiefung kann kreisförmig sein. Es kann sich bei dem Grundelement um eine Multi-Well-Platte handeln, in der mehrere Vertiefungen in einer regelmäßigen Struktur angeordnet sind. Ein Beispiel ist eine Platte mit 24 Vertiefungen, die in sechs Spalten und vier Reihen angeordnet sind. Auch eine Platte mit sechs Vertiefungen mit drei Spalten und zwei Reihen ist möglich. Gegenüber einer einzelnen Vertiefung bietet eine Multi-Well-Platte den Vorteil, dass eine Mehrzahl von Proben gleichzeitig kultiviert und der Durchsatz dadurch erhöht werden kann. Alle Vertiefungen der Multi-Well-Platte können gleich ausgebildet sein.

Der mittlere Durchmesser der Vertiefung beträgt beispielsweise 16,0 mm. Eine Tiefe der Vertiefung kann etwa 18,0 mm betragen.

Der Kultivierungseinsatz kann eine zylindrische Grundform aufweisen. Ein mittlerer Außendurchmesser des Kultivierungseinsatzes kann 10,0 mm betragen. Zwischen dem Kultivierungseinsatz und einer Innenwand der Vertiefung ergibt sich also ein Abstand von etwa 3 mm, wenn der Kultivierungseinsatz innerhalb der Vertiefung zentriert ist.

Weiterhin kann der Kultivierungseinsatz Mittel zur Befestigung umfassen, um ihn am Positionierungselement anzuordnen oder zu befestigen.

Der Boden des Kultivierungseinsatzes kann eine poröse Membran umfassen oder sein. Die Membran kann für Zellen oder Moleküle, sowie für Flüssigkeiten durchlässig sein. Die Membran kann als Gerüst oder Substrat für die Zellanhaftung dienen, wodurch Zellen auf der Membran ein- und mehrschichtige Strukturen ausbilden können.

Der Boden des Kultivierungseinsatzes kann eine Dicke zwischen 1 µm und 500 µm aufweisen. Insbesondere kann die Dicke zwischen 10 µm und 100 µm betragen. Beispielhafte Werte sind 12 µm und 50 µm.

Wird das Objektiv möglichst nah an den Boden der Vertiefung herangeführt, entspricht der minimale Abstand zwischen dem Objektiv und den Zellen auf dem Boden des Kultivierungseinsatzes der Summe aus der Dicke des Bodens der Vertiefung und der Dicke des Bodens des Kultivierungseinsatzes.

Der Kultivierungseinsatz kann in das Positionierungselement einhängbar sein, insbesondere in der ersten und/oder der zweiten Aufnahmeposition. Der Kultivierungseinsatz einen Vorsprung umfassen. Der Vorsprung kann mit einem entsprechenden Gegenstück am Positionierungselement zusammenwirken. Auf diese Weise kann der Kultivierungseinsatz in das Positionierungselement eingehängt werden.

Das Positionierungselement kann zwei unterschiedliche Ebenen aufweisen, die die erste Aufnahmeposition und die zweite Aufnahmeposition definieren, und derart ausgebildet sein, dass ein Wechsel zwischen der ersten Aufnahmeposition und der zweiten Aufnahmeposition durch eine Relativbewegung zwischen dem Positionierungselement und dem Kultivierungseinsatz erfolgt.

Der Wechsel zwischen den beiden Aufnahmepositionen erfolgt durch eine einfache Relativbewegung. Insbesondere kann das Positionierungselement ortsfest bezüglich des Grundelements sein. Der Kultivierungseinsatz kann derart ausgebildet sein, dass der Wechsel zwischen den beiden Aufnahmepositionen durch eine Bewegung des Kultivierungseinsatzes erfolgt, wobei die Bewegung eine Versetzung (translatorisch) und/oder ein Anheben bzw. Absenken und/oder eine Rotation umfasst.

Beispielsweise können die beiden Ebenen an einer oberen Kante der Vertiefung ausgebildet und durch eine Stufe verbunden sein. Hier würde ein Wechsel von der ersten Aufnahmeposition in die zweite Aufnahmeposition durch eine Rotation des Kultivierungseinsatzes in Kombination mit einem Anheben desselben über die Stufe zwischen den beiden Ebenen erfolgen.

Zwischen den beiden Ebenen kann eine Schräge vorgesehen sein, die beide Ebenen verbindet. Ein Steigungswinkel der Schräge kann größer als 0° und kleiner als 90° sein. Insbesondere kann der Steigungswinkel zwischen 20° und 70°, zwischen 30° und 60° oder zwischen 40° und 50° betragen. Der Kultivierungseinsatz muss dank der Schräge nicht angehoben werden, um in die zweite Aufnahmeposition gebracht zu werden. Während der Rotation wird die Höhendifferenz zwischen den beiden Aufnahmepositionen durch Hinaufschieben über die Schräge überwunden.

Das Positionierungselement kann bezüglich der Grundplatte beweglich und in eine erste Position und eine zweite Position bringbar sein, wobei die erste Position die erste Aufnahmeposition und die zweite Position die zweite Aufnahmeposition definieren. Damit ist das Positionierungselement in eine erste Position und eine zweite Position bezüglich des Grundelements bringbar. Die erste Position des Positionierungselements definiert die erste Aufnahmeposition für den Kultivierungseinsatz. Die zweite Position des Positionierungselements definiert die zweite Aufnahmeposition für den Kultivierungseinsatz. Daher sind die erste Position und die zweite Position unterschiedlich. Insbesondere kann das Grundelement zwei Ebenen aufweisen, die die erste Position und zweite Position für das Positionierungselement definieren.

Dies stellt eine alternative Möglichkeit dar, einen einfachen Wechsel von der ersten in die zweite Aufnahmeposition zu implementieren. Während im vorherigen Beispiel das Positionierungselement insbesondere ortsfest bezüglich dem Grundelement ist und der Wechsel durch eine Bewegung des Kultivierungseinsatzes erfolgt, wird nun das Positionierungselement bezüglich des Grundelements bewegt.

Insbesondere kann der Kultivierungseinsatz ortsfest bezüglich des Positionierungselements sein. Dies hat den besonderen Vorteil, dass der Kultivierungseinsatz nicht separat bewegt werden muss. Dies reduziert das Risiko einer versehentlichen Kontamination der Proben bzw. Zellen im Kultivierungseinsatz, das sonst durch den Kontakt zwischen dem Kultivierungseinsatz und einem Anwender zum Bewegen des Kultivierungseinsatzes gegeben sein könnte.

Die Bewegung des Positionierungselements zwischen den beiden Aufnahmepositionen kann eine rein vertikale Bewegung (parallel zu einer Längsachse der Vertiefung) sein. Alternativ kann die Bewegung des Positionierungselements eine Kombination aus einer horizontalen und einer vertikalen Bewegung sein. Ein besonderer Vorteil der rein vertikalen Bewegung besteht darin, dass es keine engen Beschränkungen hinsichtlich der Höhendifferenz zwischen der ersten Aufnahmeposition und der zweiten Aufnahmeposition gibt. Bei einer horizontalen Bewegung wird die Reichweite stattdessen durch einen ursprünglichen Abstand zwischen der Seitenwand der Vertiefung und dem Kultivierungseinsatz beschränkt.

Erfindungsgemäß stellt das Positionierungselement die beiden Aufnahmepositionen bereit. Dabei kann das Positionierungselement selbst Mittel aufweisen, um den Kultivierungsansatz in zwei verschiedenen Anordnungspositionen am Positionierungselement anzuordnen um die beiden Aufnahmepositionen zu realisieren. Alternativ kann das Positionierungselement in zwei unterschiedliche Positionen bezüglich des Grundelements bringbar sein und auf diese Weise die beiden Aufnahmepositionen bereitstellen. Der Vorteil erhöhter Anwenderfreundlichkeit wird durch diese beiden Konfigurationen gleichermaßen erzielt.

Die Vorrichtung kann weiterhin eine Rampe umfassen, wobei das Positionierungselement von der ersten Position über die Rampe in die zweite Position bringbar ist. Eine Rampe bezeichnet in diesem Zusammenhang eine Fläche, die gegenüber einer Horizontalen um einen Winkel größer als 0° und kleiner als 90° geneigt ist. Dadurch lässt sich durch ein Schieben über die Rampe eine Höhendifferenz überwinden. Hier ist dies die Höhendifferenz zwischen der ersten Position und der zweiten Position des Positionierungselements. Der Winkel der Rampe zwischen 20° und 70°, zwischen 30° und 60° oder zwischen 40° und 50° liegen.

Dank der Rampe kann das Positionierungselement allein durch eine seitlich wirkende Kraft von der ersten Position in die zweite Position verschoben werden. Der notwendige vertikale Versatz des Positionierungselements erfolgt durch das Hinaufschieben über die Rampe. Dadurch wird der Wechsel zwischen den beiden Positionen und damit zwischen den beiden Aufnahmepositionen für den Kultivierungseinsatz deutlich vereinfacht. Überdies ist es in einfacherer Weise möglich, den Wechsel zu automatisieren. Ein entsprechend ausgebildeter Roboter muss zur Ausführung des Wechsels lediglich ausgebildet sein, das Positionierungselement seitlich zu verschieben. Dies lässt sich einfacher implementieren als ein Anheben oder eine Kombination aus Anheben und seitlicher Verschiebung.

Die Rampe kann ein Teil des Grundelements sein. Das Positionierungselement kann mit einem Teil das Grundelements in Kontakt stehen. Dieser Teil kann dann über die Rampe geschoben werden, um das Positionierungselement von der ersten in die zweite Position zu bringen. Denkbar ist beispielsweise, dass das Positionierungselement einen Standfuß umfasst, mit dem/der das Positionierungselement auf dem Grundelement steht, wobei der Standfuß über die Rampe geschoben werden kann.

Die Rampe kann auf einem horizontalen Flächenabschnitt des Grundelements angeordnet sein. Die Kante der Vertiefung kann angefast sein. Dadurch wird die Kante abgeschrägt und kann ihrerseits eine Rampe darstellen, über die der Standfuß gleiten kann.

Das Positionierungselement kann über die Rampe auch von der zweiten Position in die erste Position gebracht werden bzw. bringbar sein.

Die Rampe kann alternativ ein zusätzliches bewegliches Element der Vorrichtung sein, das bezüglich des Grundelements verschiebbar ist, wobei das Positionierungselement durch Verschieben der Rampe von der ersten Position in die zweite Position bringbar ist. Die Rampe kann lediglich horizontal verschiebbar sein.

Angesichts dessen, dass die Rampe ein zusätzliches Element ist, wird dieses auch als Rampenelement bezeichnet. Für einen Wechsel zwischen der ersten Aufnahmeposition und der zweiten Aufnahmeposition ist lediglich ein Verschieben (horizontal, keine vertikale Bewegung) des Rampenelements nötig, wobei das Verschieben einen Wechsel zwischen der ersten Position und zweiten Position des Positionierungselements bereitstellt. Insbesondere ist es nicht nötig, das Positionierungselement aktiv zu bewegen, was eine weitere Vereinfachung des Wechsels zwischen den beiden Aufnahmepositionen mit sich bringt.

Die durch das Verschieben der Rampe hervorgerufene Bewegung des Positionierungselements kann ausschließlich vertikal, also entlang einer Längsachse der Vertiefung, erfolgen. Mit anderen Worten, eine Bewegungsrichtung der Rampe und eine Bewegungsrichtung des Positionierungselements können senkrecht aufeinander stehen.

Die Vorrichtung kann eine Mehrzahl an Rampenelementen umfassen, wobei jedes Rampenelement bezüglich des Grundelements verschiebbar ist. Jedes Rampenelement kann separat verschiebbar sein, also unabhängig von den anderen Rampenelementen.

In einer Vorrichtung mit einer Rampe kann das Positionierungselement oberhalb des Grundelements angeordnet sein, wobei die Rampe zwischen dem Grundelement und dem Positionierungselement angeordnet ist, und wobei das Positionierungselement mit der Rampe in Kontakt steht. Insbesondere kann das Positionierungselement teilweise auf der Rampe aufliegen. Die Rampe kann direkt auf dem Grundelement angeordnet sein.

Diese Anordnung, bei der Grundelement, Rampe bzw. Rampenelement und Positionierungselement in dieser Reihenfolge übereinander angeordnet sind, bietet den Vorteil, dass der Wechsel zwischen den beiden Positionen ohne hohen Kraftaufwand möglich ist. Genau genommen wird die benötigte Kraft im Wesentlichen durch die Masse des Positionierungselements bestimmt.

Das Positionierungselement kann über dem Grundelement angeordnet sein und einen Standfuß mit einer zumindest teilweise abgeschrägten Unterseite aufweisen, wobei der abgeschrägte Teil der Unterseite des Standfußes mit einer Kante der Vertiefung in Kontakt steht und teilweise in die Vertiefung hineinragt, wenn sich das Positionierungselement in der ersten Position befindet. Alternativ kann das Grundelement neben der Vertiefung eine Senke umfassen, wobei die abgeschrägte Unterseite des Standfußes in die Senke hineinragt, wenn sich das Positionierungselement in der ersten Position befindet. Ein Vorteil der Ausnutzung der Vertiefung besteht darin, dass das Positionierungselement mit einer herkömmlichen Multi-Well-Platte verwendet werden kann. Eine zusätzliche Senke ist dann nicht erforderlich.

Hier wird die Rampe durch die abgeschrägte Unterseite des Standfußes implementiert. Eine Verschiebung des Positionierungselements führt dazu, dass die abgeschrägte Unterseite über die Kante der Vertiefung gleitet, wodurch sich ein Abstand zwischen dem Grundelement und dem darüber angeordneten Positionierungselement ändert. Auf diese Weise wird das Positionierungselement von der ersten Position in die zweite Position gebracht und umgekehrt. Weder ein separates Rampenelement, noch eine Rampe als Teil des Grundelements sind in diesem Fall nötig. Daher stellt diese Ausführung eine einfache und leicht herzustellende Möglichkeit dar, den Wechsel zwischen beiden Aufnahmepositionen für den Kultivierungseinsatz zu ermöglichen. Bei dieser Realisierung setzt sich die Bewegung des Positionierungselements aus einer horizontalen Verschiebung und einem vertikalen Anheben bzw. Absenken zusammen.

Ein erster Abschnitt der Unterseite kann abgeschrägt sein und ein zweiter Abschnitt, der sich an den ersten Abschnitt anschließt, kann horizontal sein. In der zweiten Position kann der zweite Abschnitt auf der Kante der Vertiefung stehen. In der ersten Position kann, wie zuvor beschrieben, der erste Abschnitt auf der Kante der Vertiefung stehen.

Der Kultivierungseinsatz kann in der zweiten Aufnahmeposition blockierbar sein, indem ein erhöhter Kraftaufwand nötig ist, um den Kultivierungseinsatz in die erste Aufnahmeposition zu bringen. Das Positionierungselement in der zweiten Position blockierbar sein, indem ein erhöhter Kraftaufwand nötig ist, um das Positionierungselement in die erste Position zu bringen. Beispielsweise kann in der zweiten Position ein Formschluss zwischen dem Positionierungselement und dem Grundelement vorliegen. Möglich ist auch ein Formschluss zwischen dem Kultivierungseinsatz und dem Positionierungselement, wenn sich der Kultivierungseinsatz in der zweiten Aufnahmeposition befindet.

Durch dieses Blockieren wird verhindert, dass der Kultivierungseinsatz unbeabsichtigt von der zweiten Aufnahmeposition in die erste Aufnahmeposition gebracht wird (z.B. durch einen leichten Stoß gegen die Vorrichtung), oder das Positionierungselement unbeabsichtigt von der ersten Position in die zweite Position gebracht wird. Beispielsweise könnte es ohne ein Blockieren passieren, dass das Positionierungselement unbeabsichtigt die Rampe herunterrutscht.

Der erhöhte Kraftaufwand ist dabei so zu verstehen, dass eine Kraft aufgewendet werden muss, die über die Haftreibung zwischen der Rampe und dem Positionierungselement hinausgeht. Beispielsweise kann an der Rampe eine zusätzliche Erhebung vorgesehen sein, die überwunden werden muss, um das Positionierungselement von der zweiten Position in die erste Position zu bringen. Alternativ kann die Rampe einen Abschnitt umfassen, der eine höhere Reibung (vor allem eine höhere Gleitreibung) mit dem Positionierungselement aufweist. Der Abschnitt der Rampe kann eine Oberfläche aus Gummi oder einem anderen Material aufweisen, das besagte Reibung erhöht.

Die Unterseite des Standfußes des Positionierungselements kann drei Abschnitte aufweisen:
- einen ersten Abschnitt, der um einen ersten Winkel gegenüber einer Horizontalen abgeschrägt ist,
- einen zweiten Abschnitt, der an den ersten Abschnitt anschließt und um einen zweiten Winkel mit entgegengesetztem Vorzeichen zum ersten Winkel gegenüber der Horizontalen abgeschrägt ist, und
- einen dritten Abschnitt, der an den zweiten Abschnitt anschließt und parallel zur Horizontalen ausgebildet ist,
wobei in der ersten Aufnahmeposition der erste Abschnitt in Kontakt mit der Kante der Vertiefung steht, und wobei in der zweiten Aufnahmeposition der dritte Abschnitt in Kontakt mit der Kante der Vertiefung steht. Die Horizontale ist insbesondere senkrecht zur Vertikalen bzw. zur Längsachse der Vertiefung.

Durch diese drei gegebenen Abschnitte besitzt die Unterseite des Standfußes einen dreieckigen Vorsprung, der als Zahn bezeichnet werden kann. In der zweiten Position des Positionierungselements liegt der dritte Abschnitt auf der Kante der Vertiefung. Um das Positionierungselement nun in die erste Position zu bringen muss der Zahn überwunden werden, was einen erhöhten Kraftaufwand darstellt. Dadurch wird das Positionierungselement in der zweiten Position blockiert oder verrastet und kann nicht unbeabsichtigt in die erste Position hinunterfallen. Wenn der Zahn überwunden ist, rutscht das Positionierungselement entlang des ersten Abschnitts in die erste Position.

Der dritte Abschnitt kann auch um einen Winkel von höchstens 10° gegenüber der Horizontalen geneigt sein. Auch dies stellt in der zweiten Position des Positionierungselements sicher, dass der dritte Abschnitt auf der Kante der Vertiefung ruht und nicht verrutschen kann. Bei ausreichend kleinem Winkel ist die Haftreibung zwischen dem Standfuß und dem Grundelement ausreichend groß um ein Verrutschen zu vermeiden.

Diese Ausführung ist besonders geeignet, wenn das Grundelement eine Multi-Well-Platte mit benachbarten Vertiefungen ist. In der zweiten Position des Positionierungselements liegt der dritte Abschnitt dabei auf der Fläche zwischen den benachbarten Vertiefungen, die beide Vertiefungen separiert, auf und der Zahn ragt in die angrenzende Vertiefung hinein. Die Fläche zwischen benachbarten Vertiefungen wird auch als Zwischenwand bezeichnet.

Der erste Winkel kann zwischen 50° und 60°, insbesondere 54°, betragen. Der zweite Winkel kann zwischen 65° und 75°, insbesondere 70°, betragen.

Die angegebenen Werte für den ersten und zweiten Winkel haben sich hinsichtlich der Praktikabilität als optimal herausgestellt. Insbesondere bieten diese Winkel eine sichere Verrastung des Positionierungselements in der zweiten Position und es bedarf eines angemessenen Kraftaufwands um das Positionierungselement von der ersten in die zweite Position zu bringen.

Vom dritten Abschnitt aus gesehen kann eine Höhe des Zahns zwischen 0,7 und 0,9 mm, insbesondere 0,8 mm, betragen. An seiner Spitze kann der Zahn abgerundet sein. Die Rundung sorgt für eine gleichmäßigere Bewegung des Positionierungselements, wenn die Verschiebung über den Zahn erfolgt. Zudem ist die Höhe des Zahns so gewählt, dass eine zu überwindende Höhendifferenz nicht zu groß ist. Der dritte Abschnitt kann eine Länge, vom zweiten Abschnitt aus gesehen, von ca. 1 mm aufweisen.

Mit der Rampe muss das Positionierungselement nur seitlich geschoben werden, um einen Wechsel zwischen den beiden Positionen zu erzeugen. Der erste Abschnitt dient dabei als Rampe, der die seitliche Verschiebung zusätzlich in eine vertikale Bewegung umwandelt. Diese Verschiebung lässt sich relativ einfach mit einem Roboter automatisieren, da keine komplexen Bewegungsabläufe erforderlich sind.

Die Vorrichtung kann weiterhin ein seitliches Begrenzungselement oder mehrere seitliche Begrenzungselemente zur Begrenzung einer horizontalen Bewegung des Positionierungselements zwischen der ersten Position und der zweiten Position aufweisen. Das mindestens eine seitliche Begrenzungselement stellt sicher, dass das Positionierungselement nicht über die erste Position und die zweite Position hinaus verschoben werden können. Gleichzeitig werden die erste und zweite Position durch den Anschlag an das mindestens eine seitliche Begrenzungselement klar definiert, wodurch ein Benutzer die korrekte Position des Positionierungselements eindeutig erkennen kann. Auch im Rahmen der bereits diskutierten Automatisierung mit einem Roboter ist das mindestens eine seitliche Begrenzungselement hilfreich, weil es die beiden Positionen des Positionierungselements vorgibt und sie nicht präzise in der Programmierung des Roboters implementiert werden müssen.

Das mindestens eine seitliche Begrenzungselement kann am Grundelement angebracht sein. Das seitliche Begrenzungselement kann einen Rahmen bilden, der sich vom Grundelement nach oben erstreckt, wobei das Positionierungselement innerhalb des Rahmens verschoben werden kann.

Das Positionierungselement kann ein Durchgangsloch aufweisen, das für die Anordnung des Kultivierungseinsatzes ausgebildet ist, wobei das Durchgangsloch eine zusätzliche Ausbuchtung aufweist. Durch die Ausbuchtung kann die Form des Durchgangslochs von einer Kreisform oder Ellipsenform abweichen.

Die Ausbuchtung kann dazu benutzt werden, mit einer Pipette Nährmedium am Kultivierungseinsatz vorbei in die Vertiefung einzuführen, insbesondere, wenn sich der Kultivierungseinsatz in der zweiten Aufnahmeposition befindet. Dabei bietet die Ausbuchtung zusätzlichen Platz für die Pipette und vereinfacht die Zugabe von Nährmedium.

Das Positionierungselement kann zwei Standfüße mit den drei Abschnitten aufweisen. Das Positionierungselement kann vier Standfüße aufweisen, wovon zumindest zwei die drei Abschnitte aufweisen. Die übrigen Standfüße können eine plane Unterseite und/oder oder eine abgeschrägte Unterseite aufweisen. Die Anordnung der Standfüße kann bezüglich einer Mittelachse des Positionierungselements symmetrisch sein.

Das Vorsehen von vier Standfüßen erhöht die Stabilität der Vorrichtung, weil das Positionierungselement stabil auf dem Grundelement steht. Indem zwei der Standfüße mit den drei Abschnitten vorgesehen sind, wird sichergestellt, dass die Positionierungsvorrichtung nicht einseitig blockiert und ungewollt gegenüber dem Grundelement verdreht wird. Da die Standfüße mit den drei Abschnitten filigraner zu fertigen sind als Standfüße mit einer planen Unterseite oder einer abgeschrägten Unterseite vereinfacht sich die Fertigung des Positionierungselements, wenn nicht alle Standfüße die drei Abschnitte umfassen.

Das Grundelement kann einen Kunststoff umfassen oder aus diesem bestehen. Insbesondere kann es Kunststoffe wie COC (Cyclo-Olefin Copolymer), COP (Cyclo-Olefin Polymer), PC (Polycarbonat), PS (Polystyrol), PE (Polyethylen), PMMA (Polyethylmethacrylat) oder einen transparenten Thermoplast oder ein Elastomer umfassen. Das Positionierungselement kann einen Kunststoff umfassen oder aus diesem bestehen. Insbesondere kann es PS, Polypropylen (PP) oder Polyethylenterephthalat (PET) umfassen oder daraus bestehen. Die Wahl dieser Kunststoffe bietet den Vorteil, dass die beiden Elemente sterilisierbar und damit wiederverwendbar sind.

Das Grundelement und/der das Positionierungselement können ein Spritzgussteil sein. Durch die Verwendung der genannten Materialien können das Grundelement bzw. das Positionierungselement kostengünstig und in großer Stückzahl mit konstanter Qualität produziert werden. Alternativ können das Positionierungselements und/oder das Grundelement mittels 3D-Druck hergestellt sein. 3D-Druck hat den Vorteil, dass sich filigrane und komplexe Strukturen verlässlich herstellen lassen. Das Grundelement und/oder das Positionierungselement können einstückig sein.

Alternativ kann das Grundelement ein Glas umfassen oder daraus bestehen.

Das Glas oder der Kunststoff für das Grundelement kann insbesondere die Doppelbrechung und die Autofluoreszenz eines Schott-Deckglases (wie D 263 Schott Glas, Nr. 1.5H) aufweisen..

Die Vorrichtung kann einen Deckel umfassen, der auf das Grundelement aufsetzbar ist. Der Deckel kann formschlüssig aufsetzbar sein. Insbesondere befindet sich bei aufgesetztem Deckel das Positionierungselement zwischen dem Grundelement und dem Deckel. Der Deckel schirmt den Kultivierungseinsatz und die darin enthaltenen Zellen gegen externe Umwelteinflüsse ab und schützt sie so vor Kontamination.

Die vorliegende Erfindung stellt weiterhin ein System umfassend die voranstehend beschriebene Vorrichtung zur Kultivierung zellbiologischer Proben und einen Kultivierungseinsatz bereit.

Der Kultivierungseinsatz kann umfassen: einen Boden mit einer porösen Membran, die für Zellen oder Moleküle durchlässig ist; und ein Befestigungselement mittels dem der Kultivierungseinsatz am Positionierungselement angeordnet ist. Der Kultivierungseinsatz kann ein Wegwerfartikel für den Einmalgebrauch sein.

Weitere Merkmale und Vorteile der Erfindung werden nachfolgend anhand der beispielhaften Figuren erläutert. Dabei zeigen:
- Figur 1: eine erste Vorrichtung zur Kultivierung zellbiologischer Proben;
- Figur 2: eine zweite Vorrichtung zur Kultivierung zellbiologischer Proben;
- Figuren 3A, 3B: eine dritte Vorrichtung zur Kultivierung zellbiologischer Proben zusammen mit einer Detailansicht;
- Figuren 4A, 4B: eine vierte Vorrichtung zur Kultivierung zellbiologischer Proben zusammen mit einer Detailansicht;
- Figuren 5A, 5B: eine fünfte Vorrichtung zur Kultivierung zellbiologischer Proben zusammen mit einer Detailansicht;
- Figur 6: ein Positionierungselement einer sechsten Vorrichtung zur Kultivierung zellbiologischer Proben;
- Figur 7: ein Positionierungselement für eine siebte Vorrichtung zur Kultivierung zellbiologischer Proben;
- Figur 8: eine Detailansicht des Standfußes gemäß der siebten Vorrichtung zur Kultivierung zellbiologischer Proben; und
- Figuren 9A, 9B: eine Detailansicht der siebten Vorrichtung zur Kultivierung zellbiologischer Proben mit dem Positionierungselement in der ersten Position und der zweiten Position.

Im Folgenden und in den Figuren werden in den verschiedenen Ausführungsbeispielen, sofern nicht anders angegeben, die gleichen Bezugszeichen für gleiche oder entsprechende Elemente verwendet.

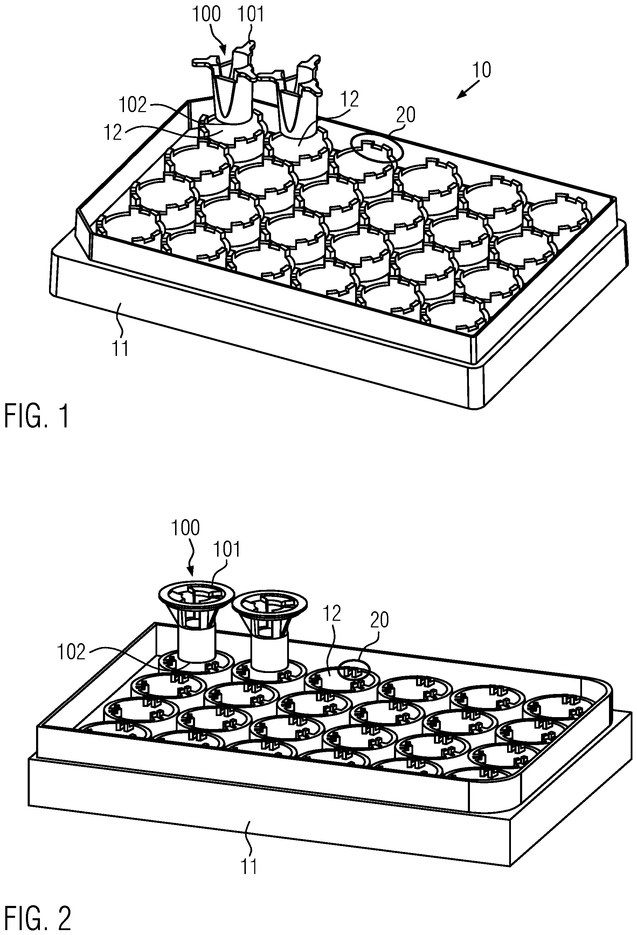

Figur 1 zeigt eine erste Vorrichtung 10 zur Kultivierung zellbiologischer Proben in einem Kultivierungseinsatz 100. Diese umfasst ein Grundelement 11, in dem eine Vertiefung 12 mit einem Boden ausgebildet ist. Insbesondere kann das Grundelement eine dargestellte Multi-Well-Platte sein, die in diesem Beispiel 24 Vertiefungen aufweist, die in einem quadratischen Gitter aus sechs Spalten und vier Reihen angeordnet sind. Alle Vertiefungen der Multi-Well-Platte sind gleich ausgebildet. Es versteht sich, dass das Grundelement 12 nicht auf die gezeigte Form und Zahl der Vertiefungen beschränkt ist.

Im Folgenden werden eine Vertiefung 12 und ein darin angeordneter Kultivierungseinsatz 100 diskutiert, wobei die dortigen Ausführungen für die anderen Vertiefungen der Multi-Well-Platte in gleicher Weise gelten. Darüber hinaus wird auch in den weiteren beschriebenen Vorrichtungen gemäß den nachfolgenden Figuren von einer solchen Multi-Well-Platte als Grundelement 11 ausgegangen, ohne dies zwingend zu erwähnen.

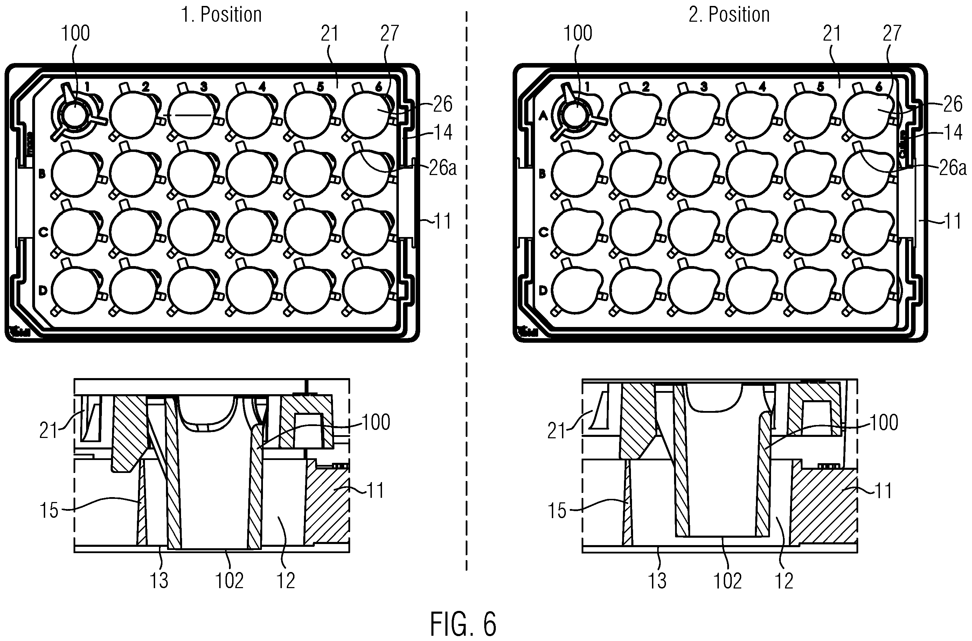

Weiterhin umfasst die Vorrichtung 10 ein Positionierungselement 20, das eine erste Aufnahmeposition und eine zweite Aufnahmeposition für den Kultivierungseinsatz 100 bereitstellt. In der ersten Aufnahmeposition ist ein Abstand zwischen dem Boden der Vertiefung 12 und einem Boden 102 des Kultivierungseinsatzes 100 kleiner ist als in der zweiten Aufnahmeposition. Eine bildliche Darstellung des Abstandes ist Figur 6 zu entnehmen, in der der Kultivierungseinsatz in der ersten und zweiten Aufnahmeposition dargestellt ist. Um dies zu erreichen, weist das Positionierungselement 20 zwei Ebenen auf. Genauer gesagt ist das Positionierungselement 20 um die Vertiefung herum als eine Struktur ausgebildet, die zwei Ebenen umfasst. Die höher gelegene Ebene definiert die zweite Aufnahmeposition, die tiefer gelegene Ebene definiert die erste Aufnahmeposition. Das Positionierungselement 20 ist dabei ortsfest gegenüber dem Grundelement 11.

Der Kultivierungseinsatz 100 kann in der Vertiefung 12 angeordnet werden, sodass er in die Vertiefung 12 hineinragt. Dazu umfasst der Kultivierungseinsatz 100 drei Befestigungselemente 101, mit denen er am Positionierungselement angeordnet an drei Auflagepunkten werden kann. Das Anordnen kann insbesondere ein Befestigen oder Einhängen sein. Im gezeigten Beispiel ist das Befestigungselement ein Vorsprung oder Haken, mit dem der Kultivierungseinsatz 100 am Positionierungselement 20 eingehängt werden kann. In der zweiten Aufnahmeposition wird der Kultivierungseinsatz 100 an der höhere gelegenen Ebene eingehängt. In der ersten Aufnahmeposition wird der Kultivierungseinsatz 100 an der tiefer gelegenen Ebene eingehängt.

Die erste Aufnahmeposition kann eine Position sein, in der der Abstand zwischen dem Boden des Kultivierungseinsatzes und dem Boden der Vertiefung möglichst klein ist. Insbesondere beträgt besagter Abstand in der ersten Aufnahmeposition null, d.h. der Boden des Kultivierungseinsatzes liegt auf dem Boden der Vertiefung auf. Möglich ist auch ein Abstand von weniger als 1 mm. In der zweiten Aufnahmeposition ist der Abstand zwischen dem Boden der Vertiefung und dem Boden des Kultivierungseinsatzes größer. Dadurch kann eine Nährlösung zwischen diesem Abstand gut strömen und einen Nährstoffaustausch durch die poröse Membran sicherstellen. In diesem Abstand kann der Abstand 1 mm oder mehr, insbesondere auch 2 mm oder mehr, oder sogar 3 mm oder mehr. Deshalb kann im Folgenden die erste Aufnahmeposition auch als Mikroskopieposition bezeichnet werden. Die zweite Aufnahmeposition kann als Kultivierungsposition bezeichnet werden.

Um von der ersten Aufnahmeposition in die zweite Aufnahmeposition zu gelangen, muss der Kultivierungseinsatz 100 bezüglich des Positionierungselement 20 vertikal angehoben und bezüglich einer Längsachse der Vertiefung 12 rotiert werden. Das Positionierungselement 20 ist bezüglich des Grundelements 11 ortsfest. Hingegen genügt es nicht, den Kultivierungseinsatz 100 lediglich zu rotieren, um von der zweiten Aufnahmeposition in die erste Aufnahmeposition zu gelangen. Dies liegt daran, dass zwischen den beiden Ebenen, in denen der Kultivierungseinsatz 100 eingehängt wird, ein zusätzliche Erhöhung vorgesehen ist. Dadurch ist der Kultivierungseinsatz 100 in der zweiten Aufnahmeposition blockiert und muss zuerst angehoben werden, um ihn rotieren zu können. Dies stellt einen erhöhten Kraftaufwand gegenüber einer reinen Rotation dar und verhindert, dass der Kultivierungseinsatz 100 versehentlich in die erste Aufnahmeposition hinunterfallen kann.

Der Kultivierungseinsatz 100 umfasst einen Boden 102, der eine poröse Membran umfasst. Diese Membran ist für Zellen und Moleküle durchlässig und dient als Gerüst oder Substrat für die Zellanhaftung, wodurch Zellen ein- oder mehrschichtige Strukturen auf der Membran ausbilden können. Diese Strukturen sollen mikroskopisch untersuchbar sein. Gleichzeitig kann Nährlösung aus der Vertiefung durch die poröse Membran diffundieren und die Zellen mit Nährstoffen versorgen. Die poröse Membran weist insbesondere eine Dicke zwischen 10 µm und 100 µm, beispielsweise 12 µm oder 50 µm auf.

Jede der Vertiefungen umfasst einen Boden (siehe Figur 6), der im sichtbaren Bereich und/oder im nahinfraroten Bereich (insbesondere bis zu einer Wellenlänge von 1500 nm) transparent ist. Daher kann durch den Boden hindurch inverse Mikroskopie durchgeführt werden. Der Boden der Vertiefung 12 kann ein Deckglas oder ein(en) Kunststoff, z.B. eine Polymerfolie, umfassen oder sein. Die optischen Eigenschaften des Bodens entsprechen insbesondere jenen eines Deckglases (z.B. D 263 Schott Glas, Nr. 1.5H). Die Dicke des Bodens beträgt zwischen 100 µm und 2,0 mm, insbesondere zwischen 160 µm und 190 µm, weiter insbesondere zwischen 165 µm und 175 µm, zum Beispiel 170 µm. Ein minimaler Abstand zwischen den Zellen und einem Objektiv des Mikroskops ist damit die Summe aus der Dicke des Bodens der Vertiefung 12 und der Dicke der porösen Membran (Boden 102) des Kultivierungseinsatzes 100. Dieser Umstand vereinfacht die Verwendung der Vorrichtung 10 für Kultur und Mikroskopie bei gleichzeitig höher optischer Auflösung und verringert obendrein das Risiko einer möglichen Kontamination, das die Zellen für die Mikroskopie nicht aus der Vertiefung 12 entfernt werden müssen.

In der ersten Aufnahmeposition kann durch den geringen Abstand zwischen den Zellen und dem Objektiv hochauflösende Mikroskopie vorgenommen werden, ohne den Kultivierungseinsatz 100 aus der Vertiefung 12 entnehmen zu müssen. In der zweiten Aufnahmeposition kann hingegen Nährlösung unterhalb der porösen Membran strömen und die Zellen effektiv mit Nährlösung versorgen. Der Wechsel zwischen der ersten Aufnahmeposition und der zweiten Aufnahmeposition ist dabei besonders einfach und anwenderfreundlich.

Um zwischen den beiden Aufnahmepositionen zu wechseln ist es folglich nicht nötig, den Kultivierungseinsatz 100 aus der Vertiefung zu entfernen. Dies verringert ebenfalls ein Kontaminationsrisiko der Zellen und vereinfacht die Handhabung für einen Anwender.

Figur 2 zeigt eine zweite Vorrichtung 10, die der ersten Vorrichtung gemäß Figur 1 in weiten Teilen ähnlich ist. Auf eine erneute Beschreibung identischer Elemente wird daher verzichtet. Der wesentliche Unterschied besteht in der Ausführung des Positionierungselements 20.

Dieses ist an einer Kante der Vertiefung 12 ausgebildet und besteht aus drei Paaren von Haken, wobei die Paare regelmäßig an der Kante angeordnet sein. In der zweiten Aufnahmeposition wird der Kultivierungseinsatz 100 an den Haken eingehängt. In der ersten Aufnahmeposition wird der Kultivierungseinsatz 100 neben den Haken auf die Kante der Vertiefung 12 aufgelegt. Ebenso wie in der ersten Vorrichtung 10 gemäß Figur 1 erfolgt ein Wechsel von der ersten in die zweite Aufnahmeposition durch ein Anheben und Verdrehen des Kultivierungseinsatzes 100. Ebenso ist der Kultivierungseinsatz 100 in der zweiten Aufnahmeposition blockiert, weil die Haken kein versehentliches Rotieren des Kultivierungseinsatzes 100 erlauben und ein Hinunterfallen in die erste Aufnahmeposition dadurch nicht ohne weiteres möglich ist.

Im Allgemeinen sind das Positionierungselement 20 und der Kultivierungseinsatz 100 derart aufeinander abgestimmt, dass die beiden Aufnahmepositionen implementierbar sind. Das Positionierungselement 20 speziell an die Ausgestaltung des Kultivierungseinsatzes 100 angepasst ist.

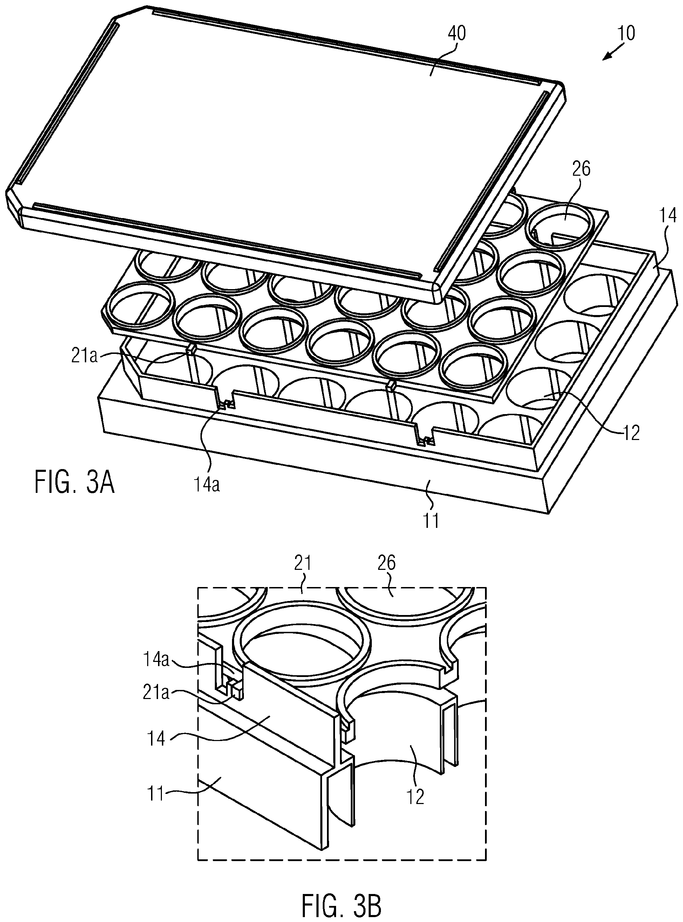

In Figur 3 ist eine dritte Form einer Vorrichtung 10 dargestellt, die eine andere Ausgestaltung des Positionierungselements 21 umfasst. Hier ist das Positionierungselement21 eine Platte, die oberhalb des Grundelements 11 angeordnet ist. Dabei ist das Positionierungselement 21 in zwei unterschiedliche Positionen bezüglich des Grundelements 11 bringbar und diese beiden Positionen definieren die zuvor eingeführte erste und zweite Aufnahmeposition für den Kultivierungseinsatz. Die erste Position des Positionierungselements 21 definiert die erste Aufnahmeposition des Kultivierungseinsatzes 100. Die erste Position des Positionierungselements 21 definiert die erste Aufnahmeposition des Kultivierungseinsatzes 100. Dies wird in Bezug auf Figur 3B genauer erläutert. Zur besseren Darstellung der einzelnen Elemente ist in Figur 3A das Positionierungselement 21 weit oberhalb des Grundelements 11 dargestellt (Explosionsansicht).

Das Positionierungselement 21 ist weiterhin ausgebildet, dass ein Kultivierungseinsatz daran angeordnet werden kann, sodass dieser in die Vertiefung 12 im Grundelement 11 hineinragt. Dazu weist das Positionierungselement ein Durchgangsloch 26 auf. Wenn das Positionierungselement 21 in der ersten Position und in der zweiten Position über dem Grundelement 11 angeordnet ist, liegt das Durchgangsloch 26 derart über der Vertiefung 12, dass der Kultivierungseinsatz durch das Durchgangsloch 26 in die Vertiefung 12 hineinragt. Insbesondere ist das Grundelement 11 eine zuvor beschriebene Multi-Well-Platte. Entsprechend weist das Positionierungselement 21 ebenso viele Durchgangslöcher 26 auf wie das Grundelement 11 Vertiefungen besitzt. Wenn das Positionierungselement 21 korrekt auf dem Grundelement 11 platziert ist, befinden sich die Durchgangslöcher 26 genau über den Vertiefungen 12.

Das Grundelement 11 weist einen nach oben weisenden und umlaufenden Rahmen auf, der als seitliches Begrenzungselement 14 dient. Dieser Rahmen schränkt die Beweglichkeit des Positionierungselements 21 insoweit ein, als dass nur die notwenige Verschiebung zwischen der ersten Position und der zweiten Position möglich ist. Anders ausgedrückt verhindert der Rahmen, dass das Positionierungselement 21 über die erste und zweite Position hinaus verschoben werden kann.

Figur 3B zeigt in einer Detailansicht, wie das Positionierungselement 21 in der zweiten Position über dem Grundelement 11 angeordnet ist. Der Rahmen weist eine seitliche Aussparung 14a auf, in der zwei Ebenen ausgebildet sind. Das Positionierungselement 21 weist einen seitlichen Vorsprung 21a auf, der in die Aussparung 14a hineinragt und auf einer der Ebenen zum Liegen kommt. In der zweiten Position liegt der Vorsprung 21a auf der höher liegenden Ebene auf. Für einen Wechsel in die erste Position (nicht gezeigt) wird das Positionierungselement 21 derart versetzt und abgesenkt, dass der Vorsprung auf der tiefer liegenden Ebene zum Liegen kommt. Umgekehrt wird das Positionierungselement 21 für einen Wechsel von der ersten in die zweite Position angehoben und versetzt.

Zwischen den beiden Ebenen der Aussparung 14a kann eine Erhöhung vorgesehen sein, die verhindert, dass das Positionierungselement 21 unbeabsichtigt in die erste Position hinunterfällt. Insbesondere kann in der zweiten Position zwischen dem Positionierungselement 21 und dem Grundelement 11 ein Formschluss hergestellt sein, indem der Vorsprung 21a zwischen der Erhöhung und einer Seitenwand der Aussparung 14a eingeklemmt ist.

Eine Eigenschaft des Positionierungselements 21 in Form der gezeigten Platte besteht darin, dass alle Kultivierungseinsätze gleichzeitig von der ersten Aufnahmeposition in die zweite Aufnahmeposition oder umgekehrt bringbar sind. In den Vorrichtungen der Figuren 1 und 2 sind die Kultivierungseinsätze einzeln von der ersten Aufnahmeposition in die zweite Aufnahmeposition oder umgekehrt bringbar werden. Je nach gewünschter Anwendung kann eine der beiden Typen des Positionierungselements 20, 21 bevorzugt sein.

Die Vorrichtung 10 kann weiterhin einen Deckel 40 umfassen, der auf das Grundelement 11 aufsetzbar ist. Der Deckel schirmt die Vertiefungen 12 gegen die Umgebung ab. Der Deckel kann formschlüssig auf das Grundelement 11 aufsetzbar sein. Das Positionierungselement 20 befindet sich insbesondere zwischen dem Grundelement 11 und dem Deckel 40. So lassen sich die Proben im Kultivierungseinsatz wirksam gegen externe Umwelteinflüsse und Kontamination abschirmen. Der Deckel kann auf alle hierin beschriebenen Vorrichtungen entsprechend aufsetzbar sein.

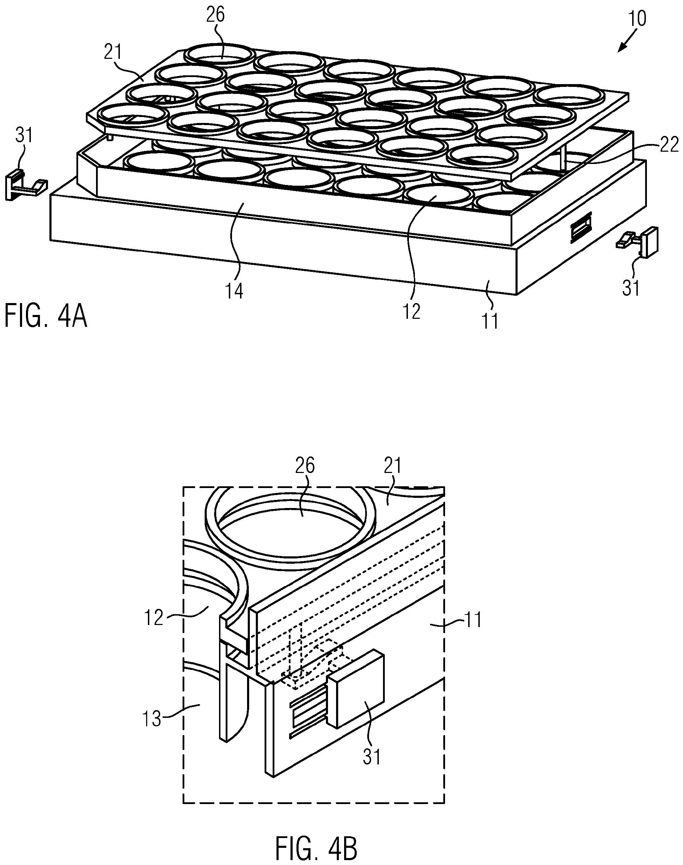

Figur 4A zeigt eine weitere Vorrichtung 10 mit einer alternativen Implementierung für den Wechsel zwischen der ersten Position und der zweiten Position. Das Grundelement 11 und Positionierungselement 21 decken sich weitgehend mit jenem aus der dritten Vorrichtung, sodass hier nur die Unterschiede erläutert werden. Zur besseren Veranschaulichung der einzelnen Komponenten ist Figur 4A eine Explosionsansicht.

Hier weist das Positionierungselement 21 an Stelle eines seitlichen Vorsprungs (siehe Figur 3B) einen Standfuß 22 auf. Dieser ist eine Säule, die sich von der Oberfläche des Positionierungselements 21 erstreckt und auf dem Grundelement 11 steht. Die Vorrichtung 10 umfasst weiterhin eine bewegliche Rampe als Rampenelement 31. Das Rampenelement 31 umfasst eine untere Ebene, die über eine Steigung mit einer oberen Ebene verbunden ist. Über den Standfuß 22 steht das Positionierungselement 21 auf dem Rampenelement 31. Wie auch in Figur 3A umfasst die Vorrichtung 10 ein seitliches Begrenzungselement 14 in Form eines nach oben weisenden, umlaufenden Rahmens, der am Grundelement 11 angeordnet ist. Da der Wechsel zwischen den beiden Positionen des Positionierungselements 21 hier rein vertikal erfolgt, kann der Rahmen sich an das Positionierungselement 21 eng anschmiegen. So werden ungewollte seitliche Verschiebungen und eine mögliche Schräglage des Positionierungselements 21 verhindert.

Figur 4B ist eine Detailansicht von Figur 4A und zeigt das Positionierungselement 21 in der ersten Position. Dabei steht der Standfuß 22 auf der unteren Ebene des Rampenelements 31. Wird das Rampenelement 31 nun verschoben, gleitet der Standfuß 22 über die Steigung hinauf auf die obere Ebene, wonach sich das Positionierungselement 21 in der zweiten Position befindet. Die vom Positionierungselement 21 dabei vollzogene Bewegung ist rein vertikal, also ohne seitlichen Versatz bezüglich des Grundelements 11.

Damit das Positionierungselement 21 zumindest in der zweiten Position nicht in Schräglage geraten kann, sind in der Vorrichtung zwei baugleiche Rampenelemente 31 vorgesehen. Diese wirken mit zwei Standfüßen 22 des Positionierungselements 21 zusammen, die auf unterschiedlichen Seiten desselben angeordnet sind. Entsprechend sind auch die beiden Rampenelemente 31 auf unterschiedlichen Seiten des Grundelements 11 angeordnet. Hierbei müssen für einen optimalen Wechsel zwischen den beiden Positionen beide Rampenelemente 31 synchron bewegt werden, da das Positionierungselement 21 sonst zwischenzeitlich in Schräglage geraten kann. Letzteres kann durch den eng angeschmiegten Rahmen weitgehend vermieden werden.

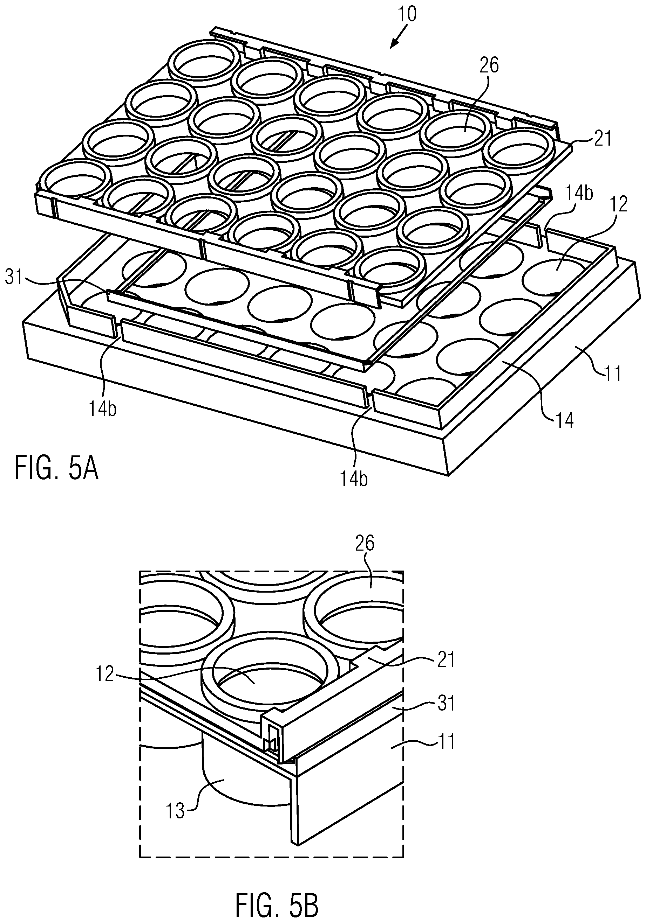

Eine Abwandlung der vierten Vorrichtung ist die fünfte Vorrichtung 10 gemäß Figuren 5A und 5B. Diese unterscheidet sich insbesondere in der Gestaltung des Rampenelements 31, die im Folgenden eingehender erklärt werden soll. Zur besseren Veranschaulichung der einzelnen Komponenten ist Figur 5A eine Explosionsansicht.

Das Rampenelement 31 hat die Form eines rechteckigen Rahmens und wird im Folgenden Positionierungsrahmen genannt. Der Positionierungsrahmen ist auf dem Grundelement 11 angeordnet wird. Das Grundelement 11 hat ähnlich wie in Figur 4A ein seitliches Begrenzungselement 14 in Form eines Rahmens, der im Übrigen die gleichen Eigenschaften und Funktionen wie zu Figur 4A erläutert, aufweist. Zusätzlich umfasst der Rahmen vier Unterbrechungen 14b, durch die der Positionierungsrahmen hindurch verläuft. Dies hat den Zweck, dass das Positionierungselement nur entlang einer Achse beweglich und ansonsten vom Rahmen blockiert ist. Auch die Reichweite dieser Bewegung wird vom Rahmen eingeschränkt. Das Positionierungselement 21 in Form einer Platte ist auf dem Rampenelement 31 positioniert.

In den Ecken des Rampenelements 31 befinden sich Schrägen, die für einen Wechsel zwischen der ersten und zweiten Position des Positionierungselements 21 sorgen (siehe Figur 4B). Dazu ist eine Oberfläche des Positionierungsrahmens im Bereich der Schrägen gegenüber einer Horizontalen angeschrägt. Auf dieser Oberfläche liegt ein entsprechender Teil des Positionierungselements. Dieser Teil kann ebenfalls mit dem gleichen Winkel angeschrägt sein, um eine formschlüssige Verbindung zwischen dem Positionierungselement 21 und dem Rampenelement zu erhalten. Alternativ kann dieser Teil auch horizontal sein.

Wird nun das Rampenelement 31 entlang der besagten Achse verschoben, gleitet das Positionierungselement 21 über die Schräge und wechselt zwischen der ersten Position und der zweiten Position. Die dabei vom Positionierungselement 21 vollzogene Bewegung ist ausschließlich vertikal. Die vom Rampenelement 31 vollzogene Bewegung ist ausschließlich horizontal.

Gegenüber der vierten Vorrichtung hat die fünfte Vorrichtung 10 den Vorteil, dass nicht gleichzeitig zwei Rampenelemente verschoben werden müssen, um zwischen den beiden Positionen zu wechseln. Allerdings kann das Rampenelement 31 hier schwieriger zu fertigen sein. Gegebenenfalls ist das Rampenelement 31 sehr filigran, wodurch Spritzguss unzuverlässig ist. 3D-Druck bietet bessere Ergebnisse.

Eine weitere Alternative zur Ausführung in Figuren 4A und 4B ist in Figur 6 dargestellt, die hier als sechste Vorrichtung 10 bezeichnet ist. Hier ist die Rampe (nicht dargestellt) ein fester Teil des Grundelements 11 und an einer Oberfläche desselben angeordnet. Das Positionierungselement 21 umfasst einen Standfuß (nicht gezeigt), der analog zum in Figur 4B geschilderten Fall über die Rampe gleitet, wenn das Positionierungselement 21 von der ersten Position in die zweite Position gebracht wird. Der Standfuß kann auch wie in Figur 4B gestaltet sein. Da die Rampe allerdings ortsfest ist, erfolgt der Wechsel zwischen den beiden Positionen durch ein Verschieben des Positionierungselements 21. Die Figur zeigt nun das Positionierungselement in der ersten Position (rechte Hälfte der Figur) und der zweiten Position (rechte Hälfte) und dazu gehörig einen Kultivierungseinsatz 100 in der ersten Aufnahmeposition (linke Hälfte) und der zweiten Aufnahmeposition (rechte Hälfte). Wie in der Figur angedeutet, ist ein Kultivierungseinsatz 100 am Positionierungselement 21 angeordnet, sodass er in die Vertiefung des Grundelements 11 hineinragt. Der Kultivierungseinsatz 100 ist bezüglich des Positionierungselements 21 ortsfest.

Gehörig zum Grundelement 11, das eine Multi-Well-Platte mit 24 Vertiefungen ist, umfasst das Positionierungselement 21 24 Durchgangslöcher 26. Diese befinden sich genau über den Vertiefungen, wenn das Positionierungselement 21 auf dem Grundelement angeordnet ist. An jedem Durchgangsloch 26 ist ein Befestigungselement 26a ausgebildet, das für die Befestigung eines Kultivierungseinsatzes mit dessen Befestigungselement 101 vorgesehen ist. Dies ist beispielhaft für ein Durchgangsloch 26 gezeigt. Dort wird der Kultivierungseinsatz 100 in das Positionierungselement 21 eingehängt, indem die Befestigungselemente 101 des Kultivierungseinsatzes 100 in das Befestigungselement 26a am Durchgangsloch eingreifen. Der Kultivierungseinsatz 100 ragt dann durch das Durchgangsloch 26 hindurch.

Zwischen zwei benachbarten Vertiefungen 12 ist eine Zwischenwand 15 ausgebildet, die die beiden Vertiefungen 12 trennt.

Jedes Durchgangsloch 26 weist eine Ausbuchtung 27 auf, wodurch die Form des Durchgangslochs 26 von einer Kreisform abweicht. Die Ausbuchtung 27 dient dazu, an dem Kultivierungseinsatz vorbei in die Vertiefung Nährlösung einzufüllen. Dies kann beispielsweise mit einer Pipette gemacht werden. Die Ausbuchtung 27 bietet dabei zusätzlichen Platz für die Pipette, was das Einfüllen von Nährlösung einfachen und komfortabler macht. Dieser Umstand ist auch in der Figur dargestellt.

Weiterhin umfasst die Vorrichtung 10 ein seitliches Begrenzungselement 14, die wie beispielsweise in Figur 3A gezeigt als umlaufender Rahmen ausgestaltet sein können. Das Positionierungselement 21 ist ausschließlich seitlich verschiebbar, wobei das Begrenzungselement 14 die seitliche Verschiebbarkeit begrenzen. Wenn das Positionierungselement 21 an der rechten Seite des Rahmens, also rechts am Begrenzungselement 14, anschlägt, ist die erste Position für das Positionierungselement 21 erreicht. Die erste Position definiert die erste Aufnahmeposition für den Kultivierungseinsatz 100, wie auch in der Figur gezeigt ist. In der ersten Aufnahmeposition steht der Boden 102 des Kultivierungseinsatzes 100 auf dem Boden 13 der Vertiefung 12 auf. Wie zuvor beschrieben stellt dies die Mikroskopieposition dar.

Wenn das Positionierungselement 21 an der linken Seite des Rahmens, also links am Begrenzungselement 14, anschlägt, ist die zweite Position für das Positionierungselement 21 erreicht. Die zweite Position definiert die zweite Aufnahmeposition für den Kultivierungseinsatz 100, wie auch in der Figur gezeigt ist. In der zweiten Aufnahmeposition hat der Boden 102 des Kultivierungseinsatzes 100 von dem Boden 13 der Vertiefung auf 12 einen Abstand, der beispielsweise 1 mm betragen kann. Wie zuvor beschrieben stellt dies die Kultivierungsposition dar.

Für den Wechsel zwischen der ersten Position und der zweiten Position muss das Positionierungselement 21 seitlich verschoben werden, wie sich aus dem Vergleich der beiden betreffenden Teilfiguren ergibt. Bei dieser Verschiebung gleitet der Standfuß über die Rampe, wodurch der vertikale Versatz zwischen der ersten Position und der zweiten Position erreicht wird.

Diese Ausführungsform hat einige Vorteile gegenüber den vorstehend beschriebenen Vorrichtungen. Einerseits kann der Wechsel zwischen den beiden Positionen 21 nur durch seitliches Verschieben des Positionierungselements 21 erfolgen und der vertikale Versatz zwischen den beiden Positionen ergibt sich automatisch durch das Hinauf- bzw. Hinunterschieben über die Rampe. Zudem gibt es für jede Richtung des Wechsels (von der ersten in die zweite Position und umgekehrt) nur einen Angriffspunkt am Positionierungselement 21. Dies macht den Wechsel automatisierbar für einen Roboter, weil dieser lediglich dazu ausgebildet sein muss, das Positionierungselement 21 in zwei entgegengesetzte Richtungen zu verschieben. Ein Abheben oder Absenken des Positionierungselements ist nicht notwendig. Weiterhin ist die Rampe als festes Element des Grundelements 11 leicht zu fertigen und nicht als separates Element der Vorrichtung 10 bereitzustellen. Dies gilt insbesondere gegenüber der fünften Vorrichtung gemäß Figur 5A, bei der das Rampenelement filigran gestaltet ist und sich nur schwer durch Spritzguss fertigen lässt.

Bei dem Grundelement 11 kann es sich um eine Multi-Well-Platte handeln. Um die Anforderungen des ANSI-SLAS-Standards für Multi-Well-Platten (z.B. betreffend Abmessungen und den Abstand zwischen benachbarten Vertiefungen/Wells) zu erfüllen, kann es notwendig sein, dass die einzelnen Vertiefungen eine elliptische Querschnittsfläche aufweisen.

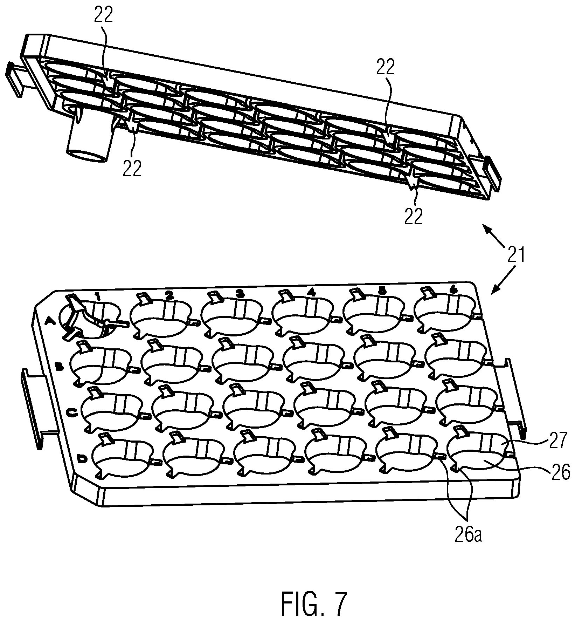

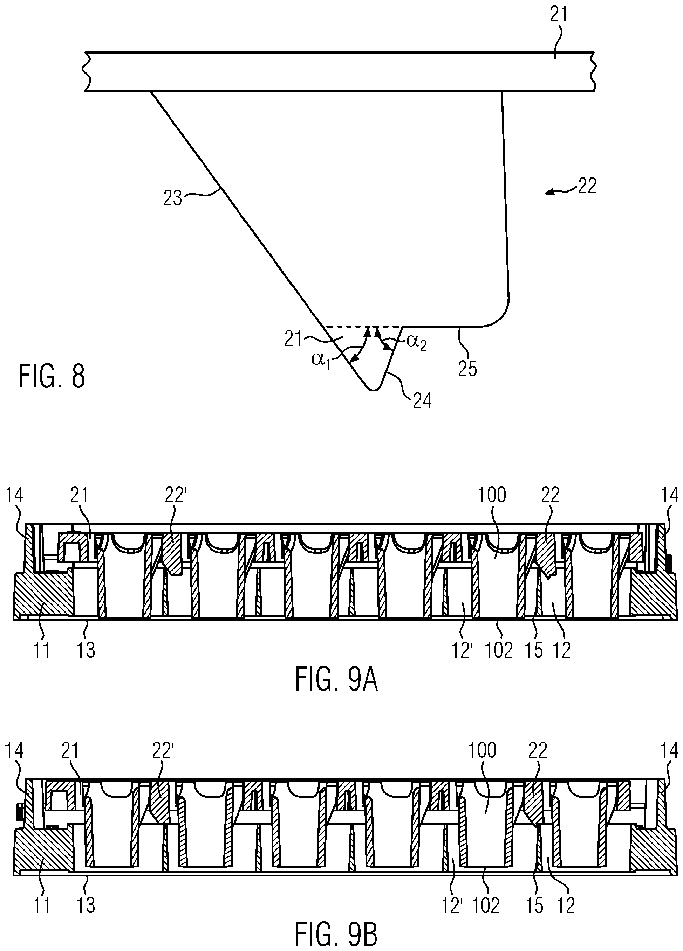

Figur 7 zeigt ein Positionierungselement 21 wie es mit einem Grundelement 11 gemäß Figur 6, allerdings ohne die Rampe, zu einer siebten Vorrichtung kombinierbar ist. Die so erhaltene Vorrichtung unterscheidet sich also von der sechsten Vorrichtung gemäß Figur 6 in der Ausgestaltung des Positionierungselements 21 und umfasst keine Rampe als Teil des Grundelements 11. Wie auch in der dritten bis sechsten vorstehend beschriebenen Vorrichtung ist das Positionierungselement 21 eine Platte, die auf dem Grundelement angeordnet werden kann. Auch die Durchgangslöcher 26 und deren Ausbuchtung 27 wurden bereits beschrieben. Ein wesentlicher Unterschied hingegen besteht in der Form des Standfußes 22, die nun in Bezug auf Figur 8 eingehender beschrieben wird. Das Positionierungselement umfasst insgesamt vier Standfüße 22. Zwei sind in der Figur direkt gezeigt, die anderen beiden befinden in gleicher Konfiguration auf der gegenüberliegenden Seite des Positionierungselements 21.

Figur 8 zeigt eine Detailansicht des zuvor beschriebenen Standfußes 22, der an einer Unterseite des Positionierungselements 21 angeordnet ist. Die Unterseite des Standfußes 22 weist drei Abschnitte auf: einen ersten Abschnitt 23, einen zweiten Abschnitt 24 und einen dritten Abschnitt 25. Der erste Abschnitt 23 ist einen Winkel α₁ von 54° gegenüber einer Horizontalen abgeschrägt bzw. geneigt. An den ersten Abschnitt 23 schließt sich der zweite Abschnitt 24 an, der um einen zweiten Winkel α₂ von 70° in entgegengesetzter Richtung zum ersten Abschnitt abgeschrägt bzw. geneigt ist. Da die Winkel in entgegengesetzter Richtung angeordnet sind, bilden die beiden Abschnitte 23, 24 eine Spitze mit einem Winkel von 56°. An den zweiten Abschnitt 24 schließt sich der dritte Abschnitt 25 an, der im Wesentlichen horizontal ausgebildet ist. Durch diese Anordnung ergibt sich vom dritten Abschnitt 25 aus gesehen eine Erhöhung, die vom zweiten 24 und ersten Abschnitt 23 gebildet wird. Diese Erhöhung wird auch als Zahn bezeichnet. Gegenüber dem dritten Abschnitt 25 hat der Zahn eine Höhe von 0,8 mm. Die Spitze des Zahns kann abgerundet sein.

Figuren 9A und 9B zeigen eine Querschnittsansicht der siebten Vorrichtung 10. Wie zuvor beschrieben, handelt es sich bei dem Grundelement 11 um eine Multi-Well-Platte mit insgesamt 24 Vertiefungen 12. In dieser Querschnittsansicht ist eine Reihe mit sechs Vertiefungen 12 gezeigt. In jeder Vertiefung ist ein Kultivierungseinsatz 100 angeordnet, der wiederum am Positionierungselement 21 eingehängt ist.

Figur 9A zeigt nun das Positionierungselement 21 in der ersten Position, wobei die Kultivierungseinsätze 100 entsprechend in der ersten Aufnahmeposition vorliegen. In dieser Aufnahmeposition stehen die Kultivierungseinsätze auf dem Boden 13 der Vertiefung 12. Anders ausgedrückt ist der Boden 102 jedes Kultivierungseinsatzes 100 in Kontakt mit dem Boden 13 der jeweiligen Vertiefung 12. Das Positionierungselement 21 schlägt an der rechten Seite an dem seitlichen Begrenzungselement 14 an, sodass das Positionierungselement 21 nicht weiter nach rechts verschiebbar ist. In dieser ersten Position steht der erste Abschnitt 23 des Standfußes 22 in Kontakt mit einer Kante der Vertiefung 12. Da das Positionierungselement 21 am seitlichen Begrenzungselement 14 anschlägt, kann es nicht weiterrutschen, obwohl der abgeschrägte erste Abschnitt 23 auf der Kante steht.

Das Positionierungselement 21 kann in der ersten Position weiter abgesenkt sein als dies durch die erste Aufnahmeposition definiert ist. Die Differenz zwischen entsprechender erster Position und tatsächlicher erster Position wird als Hub-Übermaß bezeichnet. Durch dieses Hub-Übermaß wird sichergestellt, dass alle Kultivierungseinsätze 100 in einer Multi-Well-Platte in Kontakt mit dem Boden 13 der Vertiefung 12 stehen. Eventuelle Abweichungen in den Kultivierungseinsätzen 100 oder dem Positionierungselement 21 können so ausgeglichen werden. Das Hub-Übermaß kann zwischen 0,3 mm und 0,5 mm, beispielsweise 0,4 mm, betragen.

Figur 9B zeigt nun das Positionierungselement 21 in der zweiten Position, wobei die Kultivierungseinsätze 100 entsprechend in der zweiten Aufnahmeposition vorliegen. Hier besteht ein Abstand zwischen dem Boden 13 der Vertiefung 12 und dem Boden 102 des Kultivierungseinsatzes 100. Dieser Abstand kann beispielsweise 1 mm oder mehr betragen. Das Positionierungselement 21 schlägt an der linken Seite an dem seitlichen Begrenzungselement 14 an, sodass das Positionierungselement 21 nicht weiter nach links verschiebbar ist. In der zweiten Position des Positionierungselements 21 liegt der dritte Abschnitt 25 auf der Kante der Vertiefung 12 auf. Genauer gesagt befindet sich zwischen den beiden Vertiefungen 12, 12' eine Zwischenwand 15, die beide Vertiefungen 12, 12' separiert und der dritte Abschnitt 25 liegt auf dieser Zwischenwand 15. Der Zahn ragt dabei in die benachbarte Vertiefung 12' hinein.

Um das Positionierungselement nun in die erste Position zu bringen, muss es nach rechts verschoben werden, um die Konfiguration aus Figur 9A zu erreichen. Dabei muss der Zahn von der benachbarten Vertiefung 12' in die Vertiefung 12 gebracht werden, indem der zweite Abschnitt 24 durch ein Verschieben des Positionierungselements 21 über die Kante der benachbarten Vertiefung 12' gleitet und der Zahn überwunden ist, wonach der erste Abschnitt 23 in Kontakt mit der Kante der Vertiefung 12 steht. Der Zahn und der zweite Abschnitt 24 haben folglich den Zweck, dass das Positionierungselement 21 nicht einfach von der zweiten in die erste Position wechselt, sondern ein zusätzlicher Kraftaufwand nötig ist.

Die zu Figur 8 angegebenen Winkel der Abschnitte haben sich als optimal herausgestellt. Der erste Winkel α₁ ist klein genug, um das Positionierungselement 21 unter angemessenem Kraftaufwand verschieben zu können, und groß genug, um innerhalb des zur Verfügung stehenden Raums zur seitlichen Verschiebung den Zahn zu überwinden und damit den seitlichen Versatz zwischen der ersten Position und der zweiten Position zu realisieren. Der zweite Winkel α₂ ist klein genug, um das Positionierungselement 21 über einen robotischen Antrieb zurück in die erste Position befördern zu können, und groß genug, um ein versehentliches Befördern des Positionierungselements 21 in die erste Position zu vermeiden.

Das zuvor beschriebene Konzept der Rampe für einen einfachen und automatisierbaren Wechsel zwischen den beiden Positionen des Positionierungselements ist auch in dieser Ausführungsform enthalten. Dabei dient der erste Abschnitt 23 als Rampe über die das Positionierungselement 21 hinaufgeschoben werden muss, um zwischen der ersten Position und der zweiten Position zu wechseln. Der zweite Abschnitt 24 hat den wesentlichen Zweck, das Positionierungselement 21 in der zweiten Position zu blockieren, dass es nicht versehentlich in die erste Position hinunterrutschen kann. Dennoch kann der Wechsel zwischen den beiden Positionen allein durch seitliche Verschiebung des Positionierungselements 21 bewerkstelligt werden. Dies hat den Vorteil, dass der Wechsel vergleichsweise einfach automatisierbar ist. Insbesondere kann ein entsprechend konfigurierter Roboter den Wechsel vollziehen, wobei der Roboter keine komplexen Bewegungen ausführen muss, um den Wechsel vorzunehmen. Dadurch können auch Langzeitkulturen automatisiert kultiviert und beobachtet werden. Bei solchen Langzeitkulturen müssen zwischenzeitlich mikroskopische Beobachtungen vorgenommen werden, wofür ein Wechsel von der zweiten in die erste Position erfolgen muss. Nach der Mikroskopie muss das Positionierungselement wieder in die zweite Position für die Mikroskopie zurückgebracht werden. Dies lässt sich nun vollständig automatisieren, was die Praktikabilität entsprechender Versuche und Versuchsreihen erleichtert.

In der gezeigten Querschnittsansicht hat einer der Standfüße 22 hat die besondere Struktur mit drei Abschnitten, die in Bezug auf Figur 8 genauer erläutert wurde. Ein zweiter Standfuß 22' umfasst lediglich zwei Abschnitte, die dem ersten und dritten Abschnitt gleichzusetzen sind. Insgesamt umfasst das Positionierungselement 21 vier Standfüße, wovon zwei die Struktur mit den drei Abschnitten 23, 24, 25 aufweisen. Dies genügt bereits, um die beschriebenen Vorteile zu erreichen. Da die drei Abschnitte filigran und schwer herzustellen sind, ist es vorteilhaft, nur zwei der Standfüße 22 entsprechend auszugestalten.

## Patentansprüche

1. Vorrichtung (10) zur Kultivierung zellbiologischer Proben in einem Kultivierungseinsatz (100), umfassend:
ein Grundelement (11), in dem eine Vertiefung (12) mit einem Boden (13) ausgebildet ist, wobei der Kultivierungseinsatz (100) in der Vertiefung (12) angeordnet werden kann, und ein Positionierungselement (20; 21), das eine erste Aufnahmeposition und eine zweite Aufnahmeposition für den Kultivierungseinsatz (100) bereitstellt,
wobei der Kultivierungseinsatz (100) in der ersten Aufnahmeposition und in der zweiten Aufnahmeposition am Positionierungselement (20; 21) angeordnet werden kann, sodass der Kultivierungseinsatz (100) in der Vertiefung (12) angeordnet ist, und
wobei in der ersten Aufnahmeposition ein Abstand zwischen dem Boden (13) der Vertiefung (12) und einem Boden (102) des Kultivierungseinsatzes (100) kleiner ist als in der zweiten Aufnahmeposition.

2. Vorrichtung (10) nach Anspruch 1, wobei das Positionierungselement (20) zwei unterschiedliche Ebenen aufweist, die die erste Aufnahmeposition und die zweite Aufnahmeposition definieren, und
wobei das Positionierungselement derart ausgebildet ist, dass ein Wechsel zwischen der ersten Aufnahmeposition und der zweiten Aufnahmeposition durch eine Relativbewegung zwischen dem Positionierungselement (20) und dem Kultivierungseinsatz (100) erfolgt.

3. Vorrichtung (10) nach Anspruch 1, wobei das Positionierungselement (21) bezüglich der Grundplatte (11) beweglich und in eine erste Position und eine zweite Position bringbar ist, wobei die erste Position die erste Aufnahmeposition und die zweite Position die zweite Aufnahmeposition für den Kultivierungseinsatz definieren.

4. Vorrichtung (10) nach Anspruch 3, weiterhin umfassend eine Rampe (31),
wobei das Positionierungselement (21) von der ersten Position über die Rampe (31) in die zweite Position bringbar ist.

5. Vorrichtung (10) nach Anspruch 4, wobei die Rampe (31) ein zusätzliches bewegliches Element der Vorrichtung (10) ist, das bezüglich des Grundelements (11) verschiebbar ist, und
wobei das Positionierungselement (21) durch Verschieben der Rampe (31) von der ersten Position in die zweite Position bringbar ist.

6. Vorrichtung (10) nach Anspruch 4 oder 5,
wobei das Positionierungselement (21) über dem Grundelement (11) angeordnet ist,
wobei die Rampe (31) zwischen dem Grundelement (11) und dem Positionierungselement (21) angeordnet ist, und
wobei das Positionierungselement (21) mit der Rampe (31) in Kontakt steht.

7. Vorrichtung (10) nach Anspruch 3 oder 4, wobei das Positionierungselement (21) über dem Grundelement angeordnet ist und einen Standfuß (22) mit einer zumindest teilweise abgeschrägten Unterseite aufweist,
wobei der abgeschrägte Teil der Unterseite des Standfußes (22) mit einer Kante der Vertiefung (12) in Kontakt steht und teilweise in die Vertiefung (12) hineinragt, wenn sich das Positionierungselement (21) in der ersten Position befindet.

8. Vorrichtung (10) nach einem der vorangegangenen Ansprüche, wobei der Kultivierungseinsatz (100) in der zweiten Aufnahmeposition blockierbar ist, indem ein erhöhter Kraftaufwand nötig ist, um den Kultivierungseinsatz (100) in die erste Aufnahmeposition zu bringen, und/oder
wobei das Positionierungselement (20; 21) in der zweiten Position blockierbar ist, indem ein erhöhter Kraftaufwand nötig ist, um das Positionierungselement (20; 21) in die erste Position zu bringen.

9. Vorrichtung (10) nach Anspruch 7, wobei die Unterseite des Standfußes (22) drei Abschnitte aufweist:
- einen ersten Abschnitt (23), der um einen ersten Winkel (α₁) gegenüber einer Horizontalen abgeschrägt ist,
- einen zweiten Abschnitt (24), der an den ersten Abschnitt (23) anschließt und um einen zweiten Winkel (α₂) mit entgegengesetztem Vorzeichen zum ersten Winkel (α₁) gegenüber der Horizontalen abgeschrägt ist, und
- einen dritten Abschnitt (25), der an den zweiten Abschnitt (24) anschließt und parallel zur Horizontalen ausgebildet ist,
wobei in der ersten Aufnahmeposition der erste Abschnitt (23) in Kontakt mit der Kante der Vertiefung (12) steht, und
wobei in der zweiten Aufnahmeposition der dritte Abschnitt (25) in Kontakt mit der Kante der Vertiefung (12) steht.

10. Vorrichtung (10) nach Anspruch 11, wobei der erste Winkel (α₁) zwischen 50° und 60°, insbesondere 54°, beträgt, und/oder
wobei der zweite Winkel (α₂) zwischen 65° und 75°, insbesondere 70°, beträgt.

11. Vorrichtung (10) nach einem der Ansprüche 3 bis 10, weiterhin umfassend ein seitliches Begrenzungselement (14) oder mehrere seitliche Begrenzungselemente (14) für eine Begrenzung einer horizontalen Bewegung des Positionierungselements (21) zwischen der ersten Position und der zweiten Position.

12. Vorrichtung (10) nach einem der Ansprüche 3 bis 11, wobei das Positionierungselement (21) ein Durchgangsloch (26) aufweist, das für die Anordnung des Kultivierungseinsatzes (100) ausgebildet ist, und
wobei das Durchgangloch (26) eine zusätzliche Ausbuchtung (27) aufweist, insbesondere, wobei die Form des Durchgangslochs (26) durch die Ausbuchtung (27) von einer Kreisform oder Ellipsenform abweicht.

13. Vorrichtung (10) nach Anspruch 9 oder 10, wobei das Positionierungselement (21) zwei Standfüße (22) mit den drei Abschnitten aufweist.

14. System zur Kultivierung biologischer Proben, umfassend:
eine Vorrichtung (10) nach einem der vorangegangenen Ansprüche, und
einen Kultivierungseinsatz (100).

15. System nach Anspruch 14, wobei der Kultivierungseinsatz (100) umfasst:
einen Boden (102) mit einer porösen Membran, die für Zellen oder Moleküle durchlässig ist; und
ein Befestigungselement (101) mittels dem der Kultivierungseinsatz (100) am Positionierungselement (20) angeordnet ist.

## Geänderte Patentansprüche

### Geänderte Patentansprüche gemäss Regel 137(2) EPÜ.

1. Vorrichtung (10) zur Kultivierung zellbiologischer Proben in einem Kultivierungseinsatz (100), umfassend:
ein Grundelement (11), in dem eine Vertiefung (12) mit einem Boden (13) ausgebildet ist, wobei der Kultivierungseinsatz (100) in der Vertiefung (12) angeordnet werden kann, und ein Positionierungselement (20; 21), das eine erste Aufnahmeposition und eine zweite Aufnahmeposition für den Kultivierungseinsatz (100) bereitstellt,
wobei der Kultivierungseinsatz (100) in der ersten Aufnahmeposition und in der zweiten Aufnahmeposition am Positionierungselement (20; 21) angeordnet werden kann, sodass der Kultivierungseinsatz (100) in der Vertiefung (12) angeordnet ist, und
wobei in der ersten Aufnahmeposition ein Abstand zwischen dem Boden (13) der Vertiefung (12) und einem Boden (102) des Kultivierungseinsatzes (100) kleiner ist als in der zweiten Aufnahmeposition,
wobei das Positionierungselement (21) bezüglich der Grundplatte (11) beweglich und in eine erste Position und eine zweite Position bringbar ist, wobei die erste Position die erste Aufnahmeposition und die zweite Position die zweite Aufnahmeposition für den Kultivierungseinsatz definieren.

2. Vorrichtung (10) nach Anspruch 1, weiterhin umfassend eine Rampe (31),
wobei das Positionierungselement (21) von der ersten Position über die Rampe (31) in die zweite Position bringbar ist.

3. Vorrichtung (10) nach Anspruch 2, wobei die Rampe (31) ein zusätzliches bewegliches Element der Vorrichtung (10) ist, das bezüglich des Grundelements (11) verschiebbar ist, und
wobei das Positionierungselement (21) durch Verschieben der Rampe (31) von der ersten Position in die zweite Position bringbar ist.

4. Vorrichtung (10) nach Anspruch 2 oder 3,
wobei das Positionierungselement (21) über dem Grundelement (11) angeordnet ist,
wobei die Rampe (31) zwischen dem Grundelement (11) und dem Positionierungselement (21) angeordnet ist, und
wobei das Positionierungselement (21) mit der Rampe (31) in Kontakt steht.

5. Vorrichtung (10) nach Anspruch 1 oder 2, wobei das Positionierungselement (21) über dem Grundelement angeordnet ist und einen Standfuß (22) mit einer zumindest teilweise abgeschrägten Unterseite aufweist,
wobei der abgeschrägte Teil der Unterseite des Standfußes (22) mit einer Kante der Vertiefung (12) in Kontakt steht und teilweise in die Vertiefung (12) hineinragt, wenn sich das Positionierungselement (21) in der ersten Position befindet, und
wobei die abgeschrägte Unterseite über die Kante der Vertiefung (12) gleitet, wenn das Positionierungselement (21) von der ersten Position in die zweite Position oder umgekehrt gebracht wird.

6. Vorrichtung (10) nach einem der vorangegangenen Ansprüche, wobei der Kultivierungseinsatz (100) in der zweiten Aufnahmeposition blockierbar ist, indem ein erhöhter Kraftaufwand nötig ist, um den Kultivierungseinsatz (100) in die erste Aufnahmeposition zu bringen, und/oder
wobei das Positionierungselement (20; 21) in der zweiten Position blockierbar ist, indem ein erhöhter Kraftaufwand nötig ist, um das Positionierungselement (20; 21) in die erste Position zu bringen.

7. Vorrichtung (10) nach Anspruch 5, wobei die Unterseite des Standfußes (22) drei Abschnitte aufweist:
- einen ersten Abschnitt (23), der um einen ersten Winkel (α₁) gegenüber einer Horizontalen abgeschrägt ist,
- einen zweiten Abschnitt (24), der an den ersten Abschnitt (23) anschließt und um einen zweiten Winkel (α₂) mit entgegengesetztem Vorzeichen zum ersten Winkel (α₁) gegenüber der Horizontalen abgeschrägt ist, und
- einen dritten Abschnitt (25), der an den zweiten Abschnitt (24) anschließt und parallel zur Horizontalen ausgebildet ist,
wobei in der ersten Aufnahmeposition der erste Abschnitt (23) in Kontakt mit der Kante der Vertiefung (12) steht, und
wobei in der zweiten Aufnahmeposition der dritte Abschnitt (25) in Kontakt mit der Kante der Vertiefung (12) steht.

8. Vorrichtung (10) nach Anspruch 7, wobei der erste Winkel (α₁) zwischen 50° und 60°, insbesondere 54°, beträgt, und/oder
wobei der zweite Winkel (α₂) zwischen 65° und 75°, insbesondere 70°, beträgt.

9. Vorrichtung (10) nach einem der vorangegangenen Ansprüche , weiterhin umfassend ein seitliches Begrenzungselement (14) oder mehrere seitliche Begrenzungselemente (14) für eine Begrenzung einer horizontalen Bewegung des Positionierungselements (21) zwischen der ersten Position und der zweiten Position.

10. Vorrichtung (10) nach einem der vorangegangenen Ansprüche, wobei das Positionierungselement (21) ein Durchgangsloch (26) aufweist, das für die Anordnung des Kultivierungseinsatzes (100) ausgebildet ist, und
wobei das Durchgangloch (26) eine zusätzliche Ausbuchtung (27) aufweist, insbesondere, wobei die Form des Durchgangslochs (26) durch die Ausbuchtung (27) von einer Kreisform oder Ellipsenform abweicht.

11. Vorrichtung (10) nach Anspruch 7 oder 8, wobei das Positionierungselement (21) zwei Standfüße (22) mit den drei Abschnitten aufweist.

12. System zur Kultivierung biologischer Proben, umfassend:
eine Vorrichtung (10) nach einem der vorangegangenen Ansprüche, und
einen Kultivierungseinsatz (100).

13. System nach Anspruch 12, wobei der Kultivierungseinsatz (100) umfasst:
einen Boden (102) mit einer porösen Membran, die für Zellen oder Moleküle durchlässig ist; und
ein Befestigungselement (101) mittels dem der Kultivierungseinsatz (100) am Positionierungselement (20) angeordnet ist.
